# EUROPEAN PATENT SPECIFICATION

(11) **EP 3 213 078 B1**
(45) Date of publication and mention of the grant of the patent: **05.12.2018**
(21) Application number: 15790086.1
(22) Date of filing: 30.10.2015
(51) Int. Cl.: G01N 33/569, G01N 33/574

(54) **IMMUNOLOGICAL TEST FOR THE DETECTION OF E7 ONCOPROTEINS IN BIOLOGICAL SAMPLES**
IMMUNOLOGISCHER TEST ZUM NACHWEIS VON E7-ONKOPROTEINEN IN BIOLOGISCHEN PROBEN
TEST IMMUNOLOGIQUE POUR LA DÉTECTION DES ONCOPROTÉINES E7 DANS DES ÉCHANTILLONS BIOLOGIQUES

(30) Priority: 31.10.2014 EP 14191193; 31.10.2014 US 201462073116 P
(43) Date of publication of application: 06.09.2017
(73) Proprietor: Mikrogen GmbH, 82061 Neuried (DE); Österreichische Akademie der Wissenschaften, 1010 Wien (AT)
(72) Inventor: JANSEN-DÜRR, Pidder, 6020 Innsbruck (AT); BÖCHER, Oliver, 82061 Neuried (DE); KOCH, Isabel, 81375 München (DE); SOUTSCHEK, Erwin, 83225 Berg (DE)
(74) Representative: Keller, Günter
(86) International application number: PCT/EP2015/075214
(87) International publication number: WO 2016/066785

(56) References cited:
- US-A1- 2005 142 541
- US-A1- 2007 166 699
- US-A1- 2013 029 322

## Description

Cervical cancer is one of the leading causes of cancer morbidity and mortality in women with more than 98% related to a human papilloma virus (HPV) infection origin. Infection with specific subtypes of HPV has been strongly implicated in cervical carcinoma genesis. Human papilloma viruses have circular, double-stranded DNA genomes that are approximately 8 kb in size and encode eight genes of which E6 and E7 have transforming properties. Viral E6 and E7 oncoproteins are necessary for malignant conversion. E7 plays a central role in both the viral life cycle and carcinogenic transformation (McLaughlin-Drubin et al., Virology 384 (2009), pp. 335-344). There are several different strains of HPV whereby some strains such as in particular HPV-16 and HPV-18 are known as high-risk type HVPs. On the other hand there are also HPV strains, such as HPV-6 and HPV-11 which are designated as low-risk type HPVs. Furthermore, there are several other HPV strains such as HPV-31, 33, 35, 39, 45, 51, 52, 56, 58 and 59 which bear a rather high risk for the patient. Those strains occur, however, with a lower frequency. It can be assumed that about 80% of cervical cancer worldwide are associated with only four types (16, 18, 31 and 45). In other 15% of cancer HPV types 33, 35 and 52 are detected.

US 2005/0142541 discloses a detection reagent for E6 proteins of high-risk HPVs comprising a mixture of monoclonal antibodies which specifically bind to E6 proteins of at least three different oncogenic HPV strains. US 2013/0029322 and US 2007/0166699, respectively, disclose assays for E7 proteins of the high-risk HPV types. US 2013/029322 discloses diagnostic test methods wherein polyclonal anti-high-risk HPV antibodies are used as capture antibodies. Although the teaching of this US patent application allows the detection of several high-risk strains it is, however, not possible to detect all HPV strains in one assay.

Since in different patients different HPV strains may be the cause for cervical cancer it is one object of the present invention to provide a diagnostic method whereby in one test at least 95%, preferably 99% or more of all high-risk types of HPV can be detected. The test should, however, not detect low-risk strains HPV-6 and HPV-11, respectively.

The present invention relates to a diagnostic test method for the detection of an E7 protein of a human papilloma virus in a biological sample characterized in that in a sandwich enzyme linked immunosorbent assay (ELISA) as capture antibody at least three different rabbit monoclonal antibodies which bind to at least three different epitopes are used and as detection antibody at least two different polyclonal anti E7 antibodies which are obtained by immunization with different antigens derived from different HPV types are used. The invention is further defined by the claims.

The immunological test of the present invention is based on the principle of a so-called sandwich ELISA. In the test "sandwich" the antigen can be considered as the "ham" and the capture and detection antibodies are the two sides of the roll. In a sandwich ELISA there are capture antibodies, which are usually attached to the surface of the reaction well. In the present invention the capture antibodies are monoclonal antibodies which were raised against different E7 proteins obtained preferably recombinantly from high-risk HPV strains.

There are several different techniques known in the art how monoclonal antibodies can be produced. Usually animals are immunized and antibody producing cells of the immunized animal are fused with tumor cells. Subsequently the antibody producing hybridoma cells are singled out in order to obtain a hybridoma cell line which produces only one type of monoclonal antibodies. In general most monoclonal antibodies are produced using the mouse system. It is, however, an important aspect of the present invention that the monoclonal antibodies are produced from rabbits.

In the diagnostic test the biological sample to be tested is brought into the wells which are already coated with the monoclonal capture antibodies. The biological sample is preferably a sample obtained from the cervix, preferably the epithelial cells of the cervix. Since human papilloma virus can also be involved in other cancer forms such as head and neck cancer (oropharyngial cancer) or anal cancer the biological sample can also be obtained from patients suffering from such cancers. For therapy it is important to know whether HPV and in particular high-risk strains thereof are involved in such cancer. From the diagnostic view it is desirable to detect all high-risk strains in one convenient assay.

The antigen, namely the HPV E7 protein (if present), binds to the capture antibodies. Afterwards unbound material is washed away. For the detection of the E7 protein a so-called detection antibody is used. According to the present invention the "detection antibodies" are polyclonal antibodies obtained by immunization of an animal with specific
E7 proteins. Different polyclonal antibodies are used which are obtained by immunization with different antigens.

In one preferred embodiment of the present invention the animal is immunized with recombinantly produced HPV-16 E7 in order to produce one type of polyclonal antibody. Another polyclonal antibody is obtained by immunizing the animal with recombinantly produced HPV-18 E7. In a further preferred embodiment the polyclonal antiserum is obtained by immunizing an animal with a mixture of the E7 proteins derived from different HPV types, preferably types 39, 51, 56 and 59. Alternatively also mixtures comprising E7 proteins of HPV types 33, 35 and 52 can be used. In an especially preferred embodiment a mixture of three or four different strains is used in a ratio of about 1:1:1:1 of E7 proteins of different HPV types. A mixture of 39, 51, 56 and 59 is especially preferred. The mixture contains between 20 and 30% of each of the four proteins when four proteins are used whereby necessarily 100% are obtained. Slight variations of the relationships are acceptable.

In a particularly preferred embodiment the three (or more) different polyclonal antibodies as described above are used together for the analysis of each sample.

The detection antibody is responsible for the test signal. In a preferred embodiment the polyclonal antibodies obtained from the animal are purified and biotinylated. The biotinylation allows the link of the detection antibody to a label which forms a detectable signal.

In preferred embodiments the label forms the signal which is preferably created by the action of an enzyme which converts a precursor to a product which results for example in a colour change of the reaction medium. Very frequently ELISA tests are performed on titer plates having several (e.g. 96) wells. The titer plates are part of the kit for performing the immunological test.

According to the present invention it is possible either to attach as capture antibody several different rabbit monoclonal antibodies into one single well or alternatively the rabbit monoclonal antibodies can be attached to different wells, whereby, however, for the detection of the complete results several wells coated with different monoclonal antibodies have to be evaluated together for a single biological sample.

The advantage of putting several, preferably three to five rabbit monoclonal antibodies into one well and to use also three different polyclonal detection antibodies into one well is the simplicity of the method. It is also possible to use more than five different rabbit monoclonal antibodies in one well, but when the number of different mAbs is too high the percentage of each mAb will be too low for a reliable detection.

In an alternative embodiment it is possible to foresee for one biological sample three different test wells which are coated with different combinations of rabbit monoclonal antibodies. These three test wells foreseen for one biological sample can be reacted with the same amount of antigen. For the detection of the antigen different polyclonal antibodies can be used in the three test wells. The advantage of separating the biological sample into three different test wells and performing the test with different polyclonal antibodies is that the diagnostic result may be more precise insofar as it can be better determined which specific type of HPV is detected in the biological sample.

The downside of separating the sample into three different wells is that the results have to be combined thereafter which cause an additional step. The advantage of using several different capture antibodies together with several different polyclonal antibodies as detection means in one well is that a result can be obtained easily insofar as it can be detected whether in the biological sample there is a high-risk variant of HPV or not.

According to the present invention the monoclonal antibodies are preferably obtained from rabbit. The polyclonal antibodies could also be obtained from other animals which are usually used for the production of polyclonal antibodies, namely rabbits, sheep, horses or goats whereby goats are particularly preferred.

The term "polyclonal antibodies", which are used as detection antibodies in the diagnostic test of the present invention, designates a purified fraction of antibodies obtained from the blood of an immunized animal. Usually the antigen is applied intravenously, intradermally, intramuscularly, or subcutaneously to the animal, preferably together with an adjuvant which triggers the formation of antibodies. Frequently the application of the antigen occurs three to four times whereby the time difference between each application (booster) of the antigen is 2-6 weeks. When the antibody titer has reached the desired level a large amount of blood is taken from the animal. The serum is obtained from the blood and subsequently the antibodies are separated from the serum. This can be done with suitable separation means which allow the enrichment of the antibodies (e.g. suitable columns).

The antigen which is used for the immunization of the animals and which is also used for the production of the rabbit monoclonal antibodies is usually recombinant material. Since the sequences of the different E7 proteins from different strains are known the genes coding for those sequences can be cloned in suitable expression vectors and the proteins can be expressed in suitable hosts (e.g. E.coli). After expression in the host the recombinantly produced E7 proteins are purified nearly to homogeneity. It is desired to avoid any impurities since such impurities may elicit unspecific antibodies.

One embodiment of the present invention relates to diagnostic test kits which are suitable for performing the diagnostic test of the present invention. Usually such kits contain the rabbit monoclonal antibodies which function as capture antibodies linked to a solid phase preferably in a reaction well. Alternatively, however, the capture antibodies can be linked to beads which may be made from plastic material (e.g. sterol). The detection antibody is usually contained within the test kit in a suitable form. In a preferred embodiment the detection antibody is present in a ready to use form or in a lyophilized form which can be reconstituted with suitable buffer solution.

In order to characterize the rabbit monoclonal antibodies further an epitope mapping has been performed. The monoclonal antibodies as further described in the Table 1 and their epitope sequences to which they bind are preferably used in the diagnostic test methods of the present invention and the test kits which are designed for performing the invention. Table 1 discloses also preferred consensus sequences wherein a "*" stands for an amino acid which may vary whereby the "*" stands, however, preferably for an amino acid which is disclosed at the relevant epitope sequence from which such consensus sequence is derived. The following epitopes to which the monoclonal antibodies bind have been identified:

**Table 1: Sequences to which the rabbit monoclonal antibodies bind**

| **RabMab** | **AA position** | **HPV** | **Sequence** | **Seq.ID** |
|---|---|---|---|---|
| 75-12 | | 16E7 | --PETTDLYSYEQLNDS | 1 |
| | | 33E7 | --PEPTDLYSYEQLSDS | 2 |
| | | 35E7 | LEPEATDLYSYEQLSDS | 3 |
| | | 52E7 | --PETTDLHSYEQLG-- | 4 |
| | | 58E7 | LHPEPTDLFSYEQLSDS | 5 |
| | **15**-**31** | **Consensus** | **--PE*TDL*SYEQL*DS** | 6 |
| | 16-30 | 59E7 | -NYEEVDLVSYEQLPD- | 7 |
| 42-3# | 85-98 | 16E7 | GTLGIVSPISSQKP | 8 |
| 143-7 | | 56E7 | MHGKVPTLQDVVLELTP | 9 |
| | | 18E7 | MHGPKATLQDIVLHLEP | 10 |
| | | 45E7 | --GPKATLQDIVLHLEP | 11 |
| | | 59E7 | MHGPKATLSDIVLDL-- | 12 |
| | | 58E7 | ----NPTLREYILDLHP | 13 |
| | **1-17** | **Consensus** | **MHG***TL****L*L*P** | 14 |
| 21-10 | | 16E7 | --LDLQPETTDLYSYEQLNDS | 15 |
| | | 31E7 | YVLDLQPKATDLHSYEQLPDS | 16 |
| | | 33E7 | --LDLYPEPTDLYSYEQLS-- | 17 |
| | | 35E7 | --LDLEPEATDLYSYEQ---- | 18 |
| | | 52E7 | YILDLQPETTDLHSYEQLGDS | 19 |
| | | 58E7 | --LDLHPEPTDLFSYEQLS-- | 20 |
| | **13-28** | **Consensus** | **--LDL*P**TDL*SYEQL*--** | 21 |
| 57-4 | | 16E7 | LDLQPETTDLYSYEQLNDS | 22 |
| | | 31E7 | --LQPKATDLHSYEQLPDS | 23 |
| | | 33E7 | LDLYPEPTDLYSYEQLS-- | 24 |
| | | 35E7 | LDLEPEATDLYSYEQLSDS | 25 |
| | | 52E7 | --LQPETTDLHSYEQLGDS | 26 |
| | | 58E7 | LDLHPEPTDLFSYEQLSDS | 27 |
| | **13-31** | **Consensus** | **LDL*P**TDL*SYEQL*DS** | 28 |
| | 26-40 | 56E7 | -------------EQL-DSSEDEDEDEVD | 29 |
| 58-3 | | 16E7 | --LDLQPETTDLYSYEQLNDS | 30 |
| | | 31E7 | ----LQPKATDLHSYEQLPDS | 31 |
| | | 33E7 | YVLDLYPEPTDLYSYEQLSDS | 32 |
| | | 35E7 | ----LEPEATDLYSYEQLSDS | 33 |
| | | 52E7 | ----LQPETTDLHSYEQLG-- | 34 |
| | | 58E7 | --LDLHPEPTDLFSYEQLSDS | 35 |
| | **11-31** | **Consensus** | **--LDL*P**TDL*SYEQL*DS** | 36 |
| | 26-40 | 56E7 | ---------------EQL-DSSEDEDEDEVD | 37 |
| 78-11 | 1-13 | 58E7 | MRGNNPTLREYIL | 38 |
| 80-2 | | 16E7 | EEEDEIDGPAGQAEP-- | 39 |
| | | 31E7 | ----VIDSPAGQAKPDT | 40 |
| | **31-51** | **Consensus** | **----*ID*PAGQA*P--** | 41 |
| | 93-105 | 59E7 | MDTLSFVSPLSAA | 42 |
| 84-2 | | 31E7 | -----EDVIDSPAGQAKPDT- | 43 |
| | | 33E7 | SSDED-EGLDRPDGQAQPAT- | 44 |
| | | 52E7 | -DEEDTDGVDRPDGQAEQAT- | 45 |
| | | 58E7 | SSDEDEIGLDRPDGQAQPATA | 46 |
| | **31-51** | **Consensus** | **S**ED****DRP*GQA**AT-** | 47 |
| 19-1 | | 56E7 | ----VPTLQDVVLELTP | 48 |
| | | 59E7 | MHGPKATLSDIVL---- | 49 |
| | **1-17** | **Consensus** | **----**TL*D*VL----** | 50 |
| 31-7 | | 11E7 | EQLEDSSE---DEV-DKVK------ | 51 |
| | | 16E7 | -QLNDSSEEE-DE-IDGPAGQAEP- | 52 |
| | | 18E7 | EQLSDS-EEENDE-IDGVNHQHLPA | 53 |
| | | 31E7 | -----SSDEE-D-VIDSPAGQAKP- | 54 |
| | **27-51** | **Consensus** | **EQL*DSS*EE*DEVID****Q**P-** | 55 |
| | 31-50 | 56E7 | ----SSEDEDEDEV-DHLQERPQQ- | 56 |
| | 27-51 | 68E7 | VSHEQLGDSD-DE-IDEPDHAVNHH | 57 |
| 55-11 | | 11E7 | LEDSSE---DEV-DKVD---- | 58 |
| | | 18E7 | --DS-EEENDE-IDGVNHQHL | 59 |
| 2 *consensus regions* | **13-45** | **Consensus** | **--DS*E***DE**D*V*----** | 60 |
| | | 45E7 | LHLEPQNELDPVDLLSYEQLS- | 61 |
| | | 59E7 | --LEPQN-YEEVDLVSYEQLPD | 62 |
| | | 68E7 | LELSPSNEIEPVDLVSHEQLG- | 63 |
| | | 70E7 | --LYPYNEIQPVDLV------- | 64 |
| | **13-45** | **Consensus** | **L*L*P*NE***VDL*S*EQL**** | 65 |
| 38-5^{$} | 57-69 | 31E7 | FSSQSESTLRLSV | 66 |
| 146-8 | | 11E7 | MHGRLVTLKDIVL---- | 67 |
| | | 56E7 | MHGKVPTLQDVVLELTP | 68 |
| *3 consensus regions* | **1-17** | **Consensus** | **MHG***TL*D*VL----** | 69 |
| | | 11E7 | PL TQHYQILT-SSS- | 70 |
| | | 16E7 | --RAHYNIVTFSSKS | 71 |
| | | 31E7 | --TSNYNIVTFSSQS | 72 |
| | | 58E7 | -ATANYYIVT-SSYT | 73 |
| | **49-61** | **Consensus** | **--***Y*I*TFSS**** | 74 |
| | | 18E7 | LNTLSFVS PWSASQQ | 75 |
| | | 45E7 | --TLSFVSPWSATNQ | 76 |
| | | 56E7 | --ALTVTSPLSASSN | 77 |
| | **91-105** | **Consensus** | **--*L***SP*SA***** | 78 |
| | **41-53** | 52E7 | DRPDGQAEQATSN | 79 |
| 159-1 | | 11E7 | MHGRLVTLKDIVLDLQPPD-- | 80 |
| | | 18E7 | --GPKATLQDIVLHLLEPQN- | 81 |
| *2 consensus regions* | | 56E7 | MHGKVPTLQDVVLELTPQTEI | 82 |
| | | 59E7 | MHGPKATLSDIVLDL------ | 83 |
| | **1-21** | **Consensus** | **MHG***TL*D*VL*L*****-** | 84 |
| | | 16E7 | ----------YSYEQLNDSSEEE---- | 85 |
| | | 31E7 | --LQPKATDLHSYEQLP---------- | 86 |
| | | 33E7 | LDLYPEPTDLYSYEQLSDSSDEDEGLD | 87 |
| | | 35E7 | --LEPEATDLYSYEQLSDS-------- | 88 |
| | **13-39** | **Consensus** | **--L*P**TDL*SYEQL*DSS*E*----** | 89 |
| 128-3 | | 11E7 | MHGRLVTLKDIVLDLQPPD---- | 90 |
| | | 18E7 | MHGPKATLQDIVLHLEPQN---- | 91 |
| | | 45E7 | MHGPQATLQEIVLHLEPQN---- | 92 |
| | | 56E7 | MHGKVPTLQDVVLELTPQTEIDL | 93 |
| | | 59E7 | MHGPKATLSDIVLDL-------- | 94 |
| | | 70E7 | ------TLQEIVLDLYPYN---- | 95 |
| | **1-23** | **Consensus** | **MHG***TL***VL*L*P**----** | 96 |
| 167-5 | | 11E7 | MHGRLVTLKDIVLDLQPPD---- | 97 |
| | | 18E7 | --GPKATLQDIVLHLEPQN---- | 98 |
| | | 56E7 | MHGKVPTLQDVVLELTPQTEIDL | 99 |
| | | 59E7 | MHGPKATLSDIVLDL-------- | 100 |
| | **1-23** | **Consensus** | **MHG***TL*D*VL*L*P**----** | 101 |

| | | | | |
|---|---|---|---|---|
| ^{#}Conformation epitope ^{$}Weak signal, possibly a conformation epitope | | | | |

The E7 proteins of the different HPV strains have about 98 to about 106 amino acids. The amino acid positions as provided in Table 1 refer to the consensus sequence as published by Ohlenschläger et al., Oncogene (2006), 5953-5959.

It is an important aspect of the present invention that by using several different monoclonal antibodies as capture antigens it is possible to selectively bind the antigen to be identified, namely the HPV protein E7 from different high-risk strains of HPV since the combination of the different rabbit monoclonal antibodies covers all potential epitopes occurring in the high-risk strains of HPV.

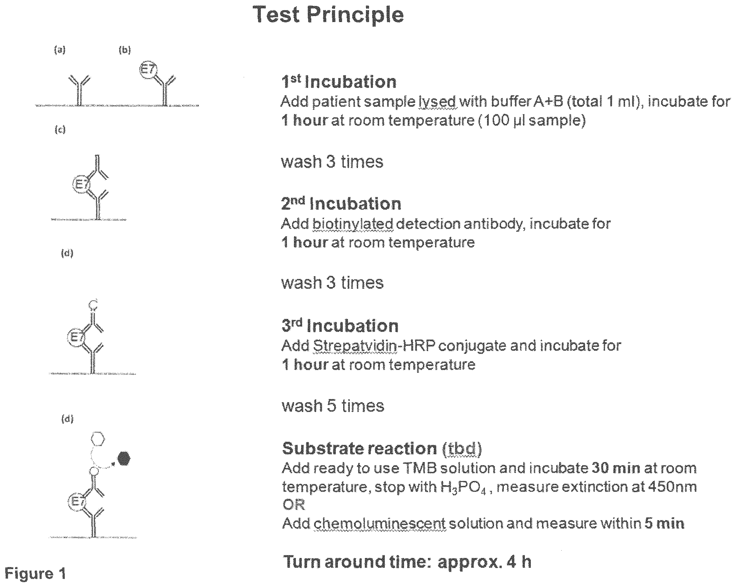

The test principle of a preferred embodiment is shown in Figure 1. For performing a diagnostic test according to the invention a suitable test kit is prepared. At the surface of the wells of the reaction holes the capture antibodies are attached to each well of the titer plate. Then a biological sample obtained from a patient is pipetted into the well. The sample is usually lysed with a special lysis buffer and incubated for a sufficient time, preferably one hour, at room temperature. This allows the binding of potential E7 antigen to the capture antibody. Subsequently the wells are washed several times, preferably three times.

In the third step the wells are incubated with the detection antibody and the reaction mixture is incubated for a sufficient time, preferably around one hour at room temperature. In order to purify the well from unbound material the well is washed preferably three to six times with a washing buffer.

In the next step the signal producing means is linked to the detection antibody. This can preferably be done by a streptavidin-biotin binding. Then the wells are washed several times in order to avoid any unspecific reaction.

Finally the signal is created usually by adding a colourless substrate which is converted by the action of the signal performing means (enzyme) into a coloured product. For example a TMB solution can be used for the development of the colour. After a certain time, usually about 30 minutes, the reaction is stopped by addition of a stopping agent (e.g. H₃PO₄) and the extinction is measured at a suitable wavelength, preferably at about 450 nm.

Preferred embodiments of the present invention are described in more detail in the examples and the figures.

### Example 1 (several capture antibodies in one well for detection of 12 hr types simultaneously)

In order to test the efficacy of different combinations of the rabbit monoclonal antibodies 75-3, 58-3, 84-2, 143-7, 159-1, and 146-8 different combinations of monoclonal antibodies were used as capture antibodies. The designation of the rabbit monoclonal antibodies correlates with the epitopes to which such antibodies bind as shown in Table 1.

**Table 2: capture antibodies present in different wells**

| **RabMab clon combination 1** | **RabMab clon combination 2** | **RabMab clon combination 3** | **RabMab clon combination 4** | **RabMab clon combination 5** | **RabMab clon combination 6** |
|---|---|---|---|---|---|
| 75-12 | 143-7 | 143-7 | 146-8 | 75-12 | 143-7 |
| 58-3 | 58-3 | 58-3 | 159-1 | 58-3 | 58-3 |
| 84-2 | 84-2 | 159-1 | - | 84-2 | 84-2 |
| - | 159-1 | - | - | 146-8 | 146-8 |
| - | - | - | - | 159-1 | 159-1 |

Afterwards, purified recombinant E7 proteins of 14 different HPV types (2 low-risk types as negative control and 12 hr types), produced in E.coli, were added to the plate in order to determine the signal pattern of each RabMab combination. Additionally, buffer without any E7 protein served as a blank (negative) control.

As detection antibodies three different polyclonal goat antibodies (short goat 1-3) were used as mixtures (goat 1+2 for combination 1 to 5, Goat 1+2+3 for combination 6). For production of the detection antibodies, different E7 proteins or combinations thereof were used as immunogen with 16E7 for goat 1, 18E7 for goat 2, and a 1:1:1:1 mixture of the E7 proteins of HPV types 39, 51, 56, and 59E7 for goat 3. The goat antibodies were biotinylated to obtain best possible sensitivity.

The detection sera, shortly goats, are summarized in Table 3.

**Table 3: detection antibodies**

| **Goats** | **final concentration in assay** | **total concentration of mixture of goat** | **Source** |
|---|---|---|---|
| Goat 1 | 0.4-1.6 µg/ml | 1.2-4.8 µm/ml | Plasma |
| Goat 2 | 0.4-1.6 µg/ml | | Plasma |
| Goat 3 | 0.4-1.6 µg/ml | | Serum |

In the test recombinant E7 proteins obtained from the strains 6, 11, 16, 18, 31, 33, 35, 39, 45, 51, 52, 56, 58, and 59 were used. The E7 proteins were produced recombinantly.

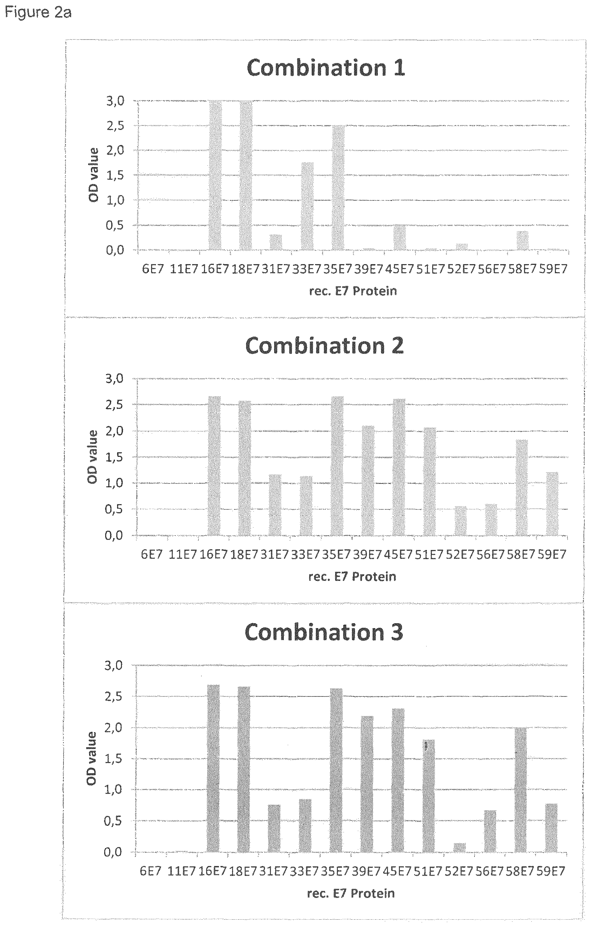

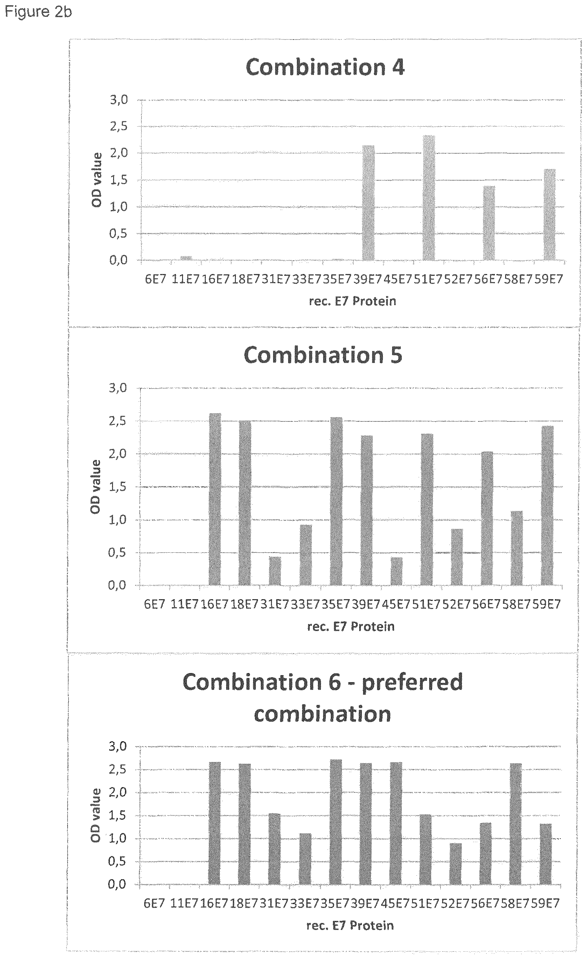

The results of the experiments can be seen in Figures 2 a)-b). The most preferred combination for detection of 12 high-risk HPV types of rabbit monoclonal antibodies is combination 6.

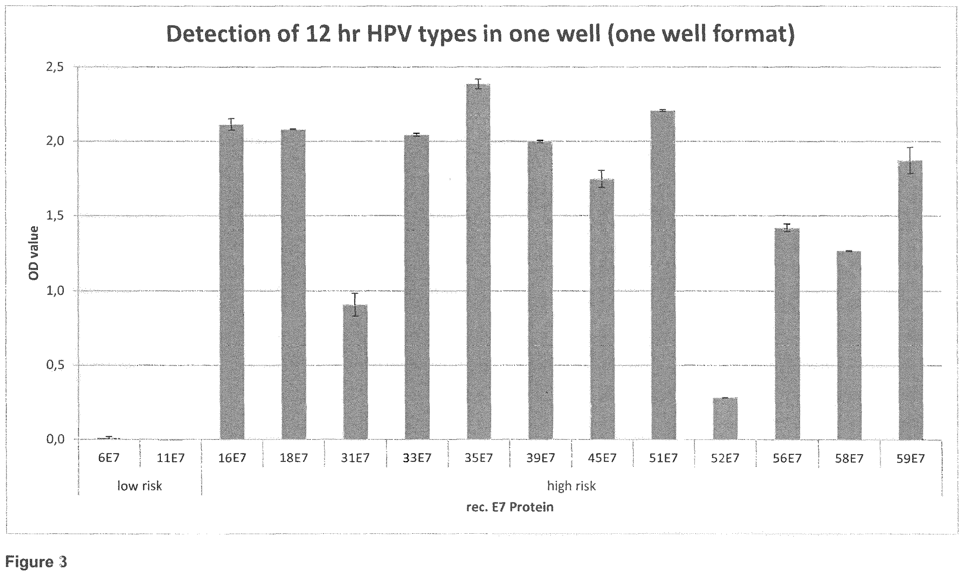

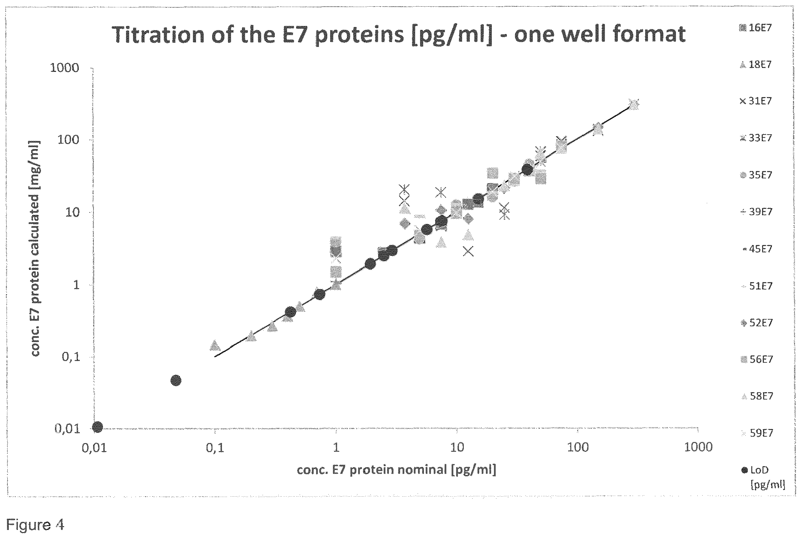

In an attempt to detect all E7 proteins in a single detection step, wells were coated with a mixture of rabbit monoclonal antibodies 143-7, 58-3, 84-2, 146-8 and 159-1. Detection was performed with a 1:1:1 mixture of biotinylated goat polyclonal antibodies goat 1, goat 2, and goat 3. With this setting the E7 oncoproteins of all high-risk HPV types analysed were detected, whereas signals obtained with low-risk HPV types 6 and 11 were in the background level. In this pan-high-risk E7 ELISA assay the detection limit varied between 0.1 picogram and 40 picogram (Figure 3 and 4) as shown with serial dilutions of all 12 hr HPV types.

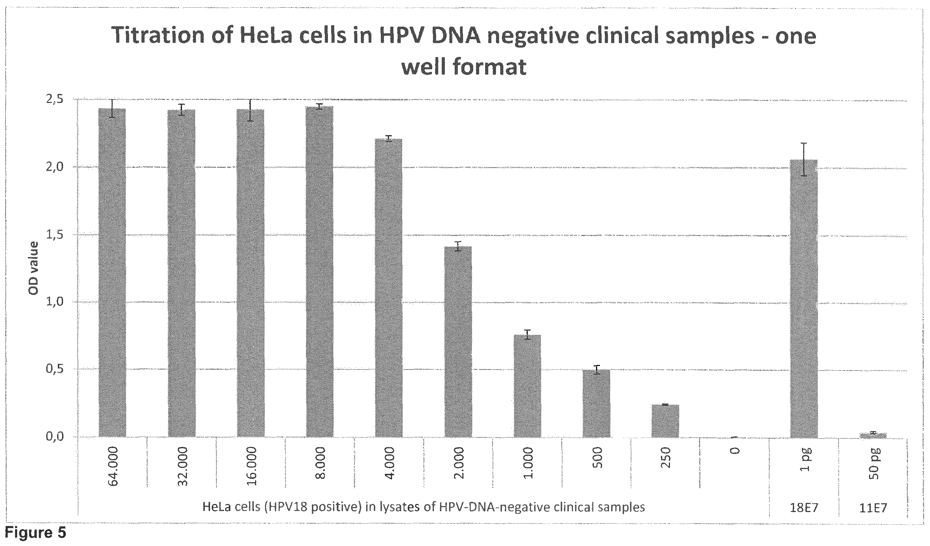

Control experiments (Figure 5) revealed signals clearly over background with 250 HeLa cells (HPV-18 positive) spiked in HPV DNA negative cervical samples from patients without clinical findings. These summarized data suggest that sufficient sensitivity is given for this format to detect 12 high-risk HPV types in one well.

### Example 2 (one sample in several reaction wells)

Rabbit monoclonal antibodies 42-3, 143-7, 58-3, 80-2, 84-2, 128-3, and 146-8 were raised against combinations of hrE7 proteins and characterized for binding specificity by direct ELISA and epitope mapping. Different combinations of rabbit monoclonal antibodies directed against various E7 proteins were used as coating antibodies on standard 96-well plates. Subsequently, purified recombinant E7 proteins of different HPV types (produced in E. coli) were added to the coated plates and used as standards for the detection sensitivity of each particular combination of RabMabs.

Bound E7 proteins were detected by the addition of affinity purified goat antibodies (referred to as Goat1, Goat2 and Goat3, respectively) raised against different E7 proteins or combinations therefore which were used as immunogens, as follows:
In all sandwich ELISA tests the signals obtained without the addition of E7 (buffer) or with the addition of low-risk E7 protein (6E7 and 11 E7) was used as negative controls.

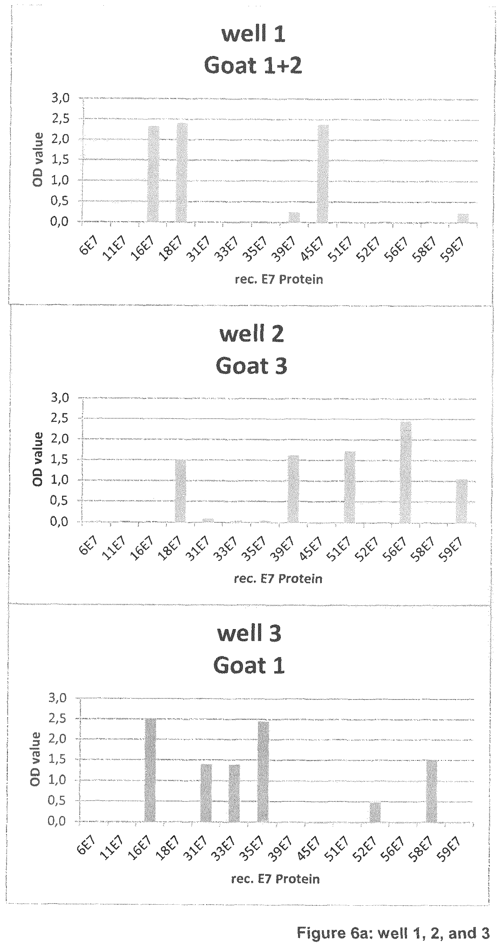

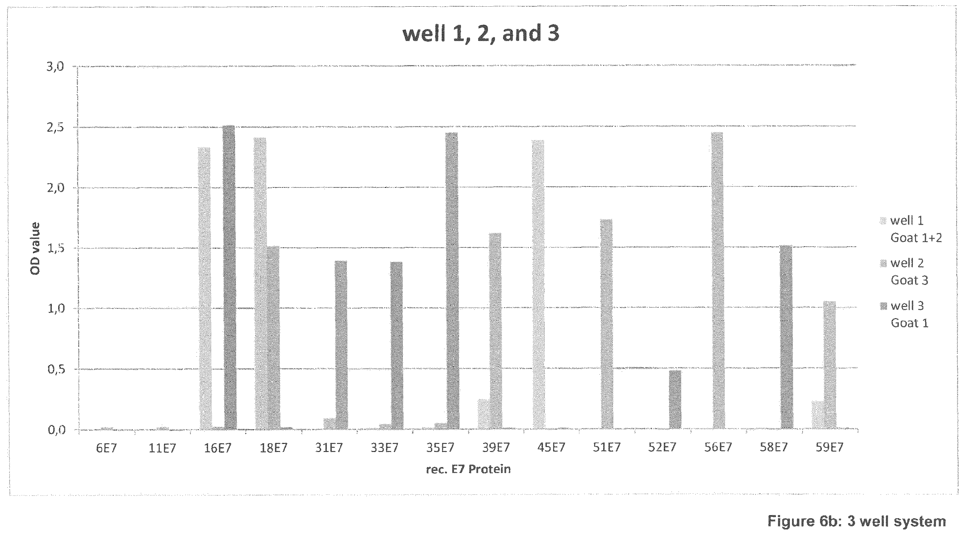

It was found that the combination (1:1) of RabMab 42-3 and 143-7 as capture antibodies, in combination with biotinylated polyclonal goat antibody goat 1 was capable to detect specifically the E7 proteins of HPV 16, HPV 18 and HPV 45(Figure 6a and b).

For the simultaneous detection of the E7 proteins of HPV type 39, 51, 56 and 59, coating was performed with a combination (1:1) of rabbit monoclonal antibodies 128-3 and 146-8. Here biotinylated antibodies Goat 3 were used. With this combination of polyclonal and monoclonal antibodies the E7 proteins of HPV types 39, 51, 56 and 59 were detected (Figure 6a and b).

For the detection of the E7 proteins encoded by HPV types 31, 33, 35, 52, and 58, a combination of rabbit monoclonal antibodies 57-4, 80-2 and 84-2 were used for coating. For detection biotinylated antibody Goat 1 was used. Under these conditions, we were able to detect the E7 proteins of HPV 31, 33, 35, 52 and 58 (Figure 6a and b).

The format of well 1 (coating antibody RabMab 42-3 and RabMab 143-7; detection with a 1:1 mixture of biotynilated goat antibody goat 1 and goat 2, see above, Fig. 6a and b) was used to determine the amount of E7 protein present in cervical cancer cell lines and in cervical smears derived from patients.

### Example 3 (control experiments)

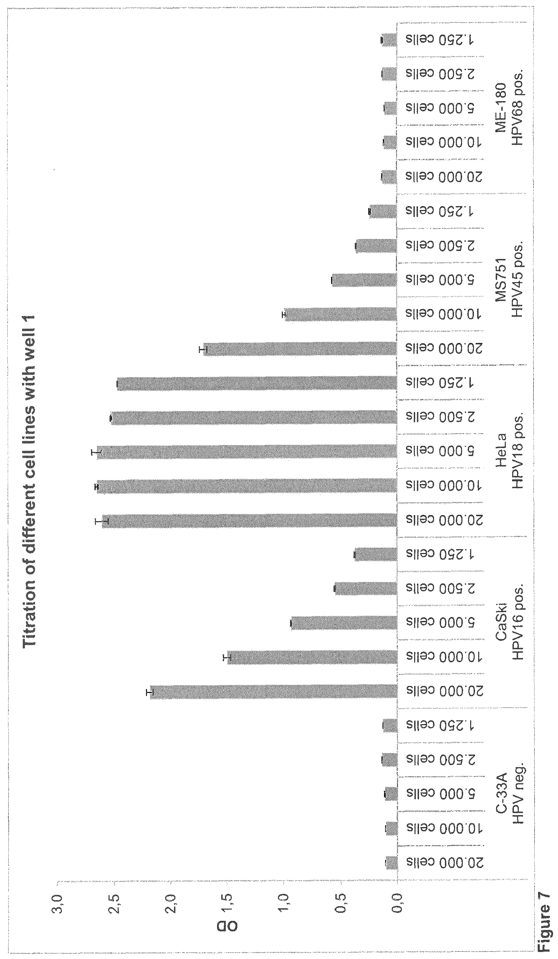

Control experiments with well 1 (Figures 7) revealed signals clearly over background with 250 HeLa cells (HPV-18 positive) or 1200 Caski cells (HPV-16 positive) or 1250 MS751 cells (HPV-45 positive). No signals were detected for the negative control cervical cancer cell lines C33a (HPV negative) as well as the HPV-68 DNA positive cell line ME-180.

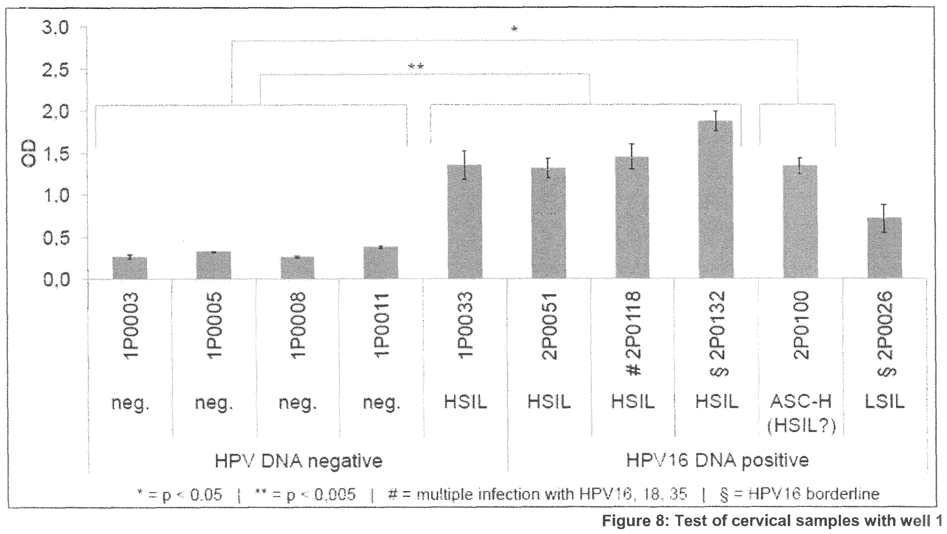

The E7 signal in HPV DNA negative samples without clinical findings was in the range of the background signal, whereas the HVP DNA positive, clinically abnormal samples, clearly displayed E7 content above background (Figure 8). These samples were characterized as CIN2 and/or above and showed high grade lesions, confirmed by histology. Furthermore, for one clinical sample which was HPV DNA negative tested but clinical abnormal with a high grade lesion, a strong positive signal for E7 was detectable. These data suggest that the setting of ELISA well 1 is already in the correct dynamic range to detect clinically abnormal smears.

### Example 4

The particularly preferred rabbit monoclonal antibodies 42-3, 143-7, 58-3, 80-2, 84-2, 128-3, and 146-8, which are used for the three well system described in Example 2 (one sample in several reaction wells), were coated together (1:1:1:1:1:1:1) in one well of standard 96-well plates. Subsequently, purified recombinant E7 proteins of 12 different hrHPV types (produced in E.coli) were added to the coated plates. Detection of bound E7 proteins was performed with a mixture of biotinylated goat polyclonal antibodies goat 1, goat 2, and goat 3 (as described in Example 2).

In order to compare the performance of the test according to the present invention with the test method as described in the prior art, different combinations of monoclonal antibodies were compared.

In well 1 (Table 4 and Table 5) the antibodies 42-3 and 143-7 as described in US 2013/0029322 were used. Furthermore, two other different combinations of monoclonal antibodies (not disclosed in prior art) were used. In addition, all monoclonal antibodies used in Example 2 were combined in one well. The arrangement of the monoclonal antibodies is shown in Table 4. For detecting the results polyclonal goat antisera was used as described in more detail in Table 5.

**Table 4**

| **RabMab-clone well 1** | **RabMab-clone well 2** | **RabMab-clone well 3** | **RabMab-clone well 1+2+3 in one well** | **final concentration in assay** | **final concentration of RabMab mixture** | **source** |
|---|---|---|---|---|---|---|
| 42-3 | 128-3 | 58-3 | 42-3 | 0.4-2 µg/ml | 2.8-10 µg/ml | Hybridom |
| 143-7 | 146-8 | 80-2 | 143-7 | 0.4-2 µg/ml | | Hybridom |
| - | - | 84-2 | 128-3 | 0.4-2 µg/ml | | Hybridom |
| - | - | - | 146-8 | 0.4-2 µg/ml | | Hybridom |
| - | - | - | 58-3 | 0.4-2 µg/ml | | Hybridom |
| - | - | - | 80-2 | 0.4-2 µg/ml | | Hybridom |
| - | - | - | 84-2 | 0.4-2 µg/ml | | Hybridom |

**Table 5**

| **goats** | **final concentration in assay** | **total concentration of goat mixture** | **source** |
|---|---|---|---|
| goat 1 | 0.4-1.6 µg/ml | 1.2-4.8 µg/ml | plasma |
| goat 2 | 0.4-1.6 µg/ml | | plasma |
| goat 3 | 0.4-1.6 µg/ml | | serum |

The setting of table 4 was tested with recombinant proteins of 12 high risk HPV types: (i) the three well system as described in example 2 (one sample in several reaction wells) with well 1 [antibodies 42-3 and 143-7 as described in US 2013/0029322], well 2 [antibodies 128-3 and 146-8] and well 3 [antibodies 58-3, 80-2, and 84-2] (both antibody combinations not disclosed in prior art), as well as (ii) all antibodies of example 2 combined in one well [antibodies 42-3, 143-7, 128-3, 146-8, 58-3, 80-2, and 84-2].

The (iii) preferred combination from example 1 (several capture antibodies in one well for detection of 12 hr types simultaneously) was tested in addition.

The results are shown in table 6: Whereas a detection of 12/12 hrHPV E7 proteins distributed over the three different wells was possible (Example 2 and column 1-3 with 4/12 hrHPV E7 proteins for well 1 [33.3%], 5/12 hr HPV E7 proteins for well 2 [41.7%], and 6/12 hrHPV E7 proteins for well 3 [50%], only 8/12 hr E7 proteins (66,7%; 16E7, 18E7, 35E7, 39E7, 45E7, 51E7, 56E7, and 59E7) were detected when the RabMabs used in the three well system of Example 2 were combined in one well (column 4). 31E7, 33E7, 52E7, and 58E7 were not detectable at all with this RabMab combination.

In the contrary, the combination used in Example 1 (several capture antibodies in one well for detection of 12 hr types simultaneously) resulted in detection of 12/12 hrHPV E7 proteins in one well (100%, column 5).

The results of this experiment can be summarized in Table 6.

**Table 6**

| | **prior art** | | | | **preferred** |
|---|---|---|---|---|---|
| **Target (HPV Subtype-E7 Protein** | **Well 1** | **Well 2** | **Well 3** | **Well 1+2+3 in one well** | **one well format** |
| | **Goat 1+2** | **Goat 3** | **Goat 1** | **Goat 1+2+3** | **Goat 1+2+3** |
| 16E7 | + | - | + | + | + |
| 18E7 | + | + | - | + | + |
| 31E7 | - | - | + | - | + |
| 33E7 | - | - | + | - | + |
| 35E7 | - | - | + | + | + |
| 39E7 | - | + | - | + | + |
| 45E7 | + | - | - | + | + |
| 51E7 | - | + | - | + | + |
| 52E7 | - | - | + | - | + |
| 56E7 | + | + | - | + | + |
| 58E7 | - | - | + | - | + |
| 59E7 | - | + | - | + | + |
| **detected/ total** | **4/12** | **5/12** | **6/12** | **8/12** | **12/12** |
| **detection [%]** | **33.3%** | **41.7%** | **50.0%** | **66.7%** | **100.0%** |

## Claims

1. Diagnostic test method for the detection of an E7 protein of a human papilloma virus in a biological sample **characterized in that** in a sandwich enzyme linked immunosorbent assay (ELISA) as capture antibody at least three different rabbit monoclonal antibodies which bind to at least three different epitopes are used and as detection antibody at least two different polyclonal anti E7 antibodies which are obtained by immunization with different antigens derived from different HPV types are used.

2. Diagnostic test method according to claim 1 **characterized in that** at least four rabbit monoclonal antibodies which bind to at least four different epitopes are used as capture antibodies.

3. Diagnostic test method according to claim 1 **characterized in that** at least five rabbit monoclonal antibodies which bind to at least five different epitopes are used as capture antibodies.

4. Diagnostic test method according to any of claims 1-3 **characterized in that** at least three different polyclonal anti E7 antibodies are used as detection antibody.

5. Diagnostic test method according to any of claims 1-4 **characterized in that** one polyclonal antibody used as detection antibody was obtained by immunization of an animal with HPV-16 E7.

6. Diagnostic test method according to any of claims 1-4 **characterized in that** one polyclonal antibody used as detection antibody was obtained by immunization of an animal with HPV-18 E7.

7. Diagnostic test method according to any of claims 1-4 **characterized in that** one polyclonal antibody used as detection antibody was obtained by immunization of an animal with a mixture of the E7 proteins of HPV types 39, 51, 56 and 59.

8. Diagnostic test method according to any of the preceding claims **characterized in that** the rabbit monoclonal antibodies are selected from the group comprising antibodies which bind to epitopes located within the following stretches of amino acids: 1-17; 11-37; 69-85; 31-51; 15-31; 85-98.

9. Diagnostic test method according to any of claims 1-8 **characterized in that** the same biological sample is tested in several reaction wells whereby each reaction well comprises at least one rabbit monoclonal antibody and the rabbit monoclonal antibodies adhered to each of said wells is different from the antibody in the other well and that each of the wells used for one sample is reacted with at least one polyclonal anti E7 antibody whereby the polyclonal anti E7 antibodies which are reacted with said biological sample are different for each well.

10. Diagnostic test method according to any of claims 1-8 **characterized in that** in one test well at least five different rabbit monoclonal antibodies which bind to at least five different epitopes are used as capture antibodies and wherein as detection antibody at least three different polyclonal anti E7 antibodies are used.

11. Diagnostic test method according to any of claims 1 to 10 **characterized in that** the biological sample is obtained from a patient suffering from cervical cancer.

12. Diagnostic test method according to any of claims 1 to 10 **characterized in that** the biological sample is obtained from a patient suffering from head and neck cancer.

13. Diagnostic test method according to any of claims 1 to 10 **characterized in that** the biological sample is obtained from a patient suffering from anal carcinoma.

14. Diagnostic test kit for performing an immunological test method according to any of claims 1-13 **characterized in that** it comprises
a solid phase coated with the monoclonal rabbit capture antibodies specified in claim 1, means for the preparation of washing solutions,
the polyclonal detecting antibodies specified in claim 1 and
means for the development of a signal.

## Patentansprüche

1. Diagnoseverfahren zum Nachweis eines E7-Proteins eines humanen Papillomavirus in einer biologischen Probe, **dadurch gekennzeichnet, dass** in einem Sandwich-heterogener-Enzym-Immunassay (Sandwich-ELISA) als Fangantikörper mindestens drei verschiedene monoklonale Kaninchen-Antikörper, die an mindestens drei verschiedene Epitope binden, verwendet werden und als Nachweisantikörper mindestens zwei verschiedene polyklonale Anti-E7-Antikörper, erhalten durch Immunisierung mit verschiedenen Antigenen, gewonnen aus verschiedenen HPV-Typen, verwendet werden.

2. Diagnoseverfahren nach Anspruch 1, **dadurch gekennzeichnet, dass** mindestens vier monoklonale Kaninchen-Antikörper, die an mindestens vier verschiedene Epitope binden, als die Fangantikörper verwendet werden.

3. Diagnoseverfahren nach Anspruch 1, **dadurch gekennzeichnet, dass** mindestens fünf monoklonale Kaninchen-Antikörper, die an mindestens fünf verschiedene Epitope binden, als die Fangantikörper verwendet werden.

4. Diagnoseverfahren nach einem der Ansprüche 1 - 3, **dadurch gekennzeichnet, dass** mindestens drei verschiedene polyklonale Anti-E7-Antikörper als Nachweisantikörper verwendet werden.

5. Diagnoseverfahren nach einem der Ansprüche 1 - 4, **dadurch gekennzeichnet, dass** ein polyklonaler Antikörper, der als Nachweisantikörper verwendet wird, durch Immunisierung eines Tiers mit HPV-16 E7 erhalten wurde.

6. Diagnoseverfahren nach einem der Ansprüche 1 - 4, **dadurch gekennzeichnet, dass** ein polyklonaler Antikörper, der als Nachweisantikörper verwendet wird, durch Immunisierung eines Tiers mit HPV-18 E7 erhalten wurde.

7. Diagnoseverfahren nach einem der Ansprüche 1 - 4, **dadurch gekennzeichnet, dass** ein polyklonaler Antikörper, der als Nachweisantikörper verwendet wird, durch Immunisierung eines Tiers mit einem Gemisch aus den E7-Proteinen der HPV-Typen 39, 51, 56 und 59 erhalten wurde.

8. Diagnoseverfahren nach einem der vorhergehenden Ansprüche, **dadurch gekennzeichnet, dass** die monoklonalen Kaninchen-Antikörper ausgewählt sind aus der Gruppe, umfassend Antikörper, die an Epitope binden, die sich in den folgenden Aminosäuresträngen befinden: 1 - 17; 11 - 37; 69 - 85; 31 - 51; 15 - 31; 85 - 98.

9. Diagnoseverfahren nach einem der Ansprüche 1 - 8, **dadurch gekennzeichnet, dass** dieselbe biologische Probe in mehreren Reaktionswells getestet wird, wobei jedes Reaktionswell mindestens einen monoklonalen Kaninchen-Antikörper umfasst und die an den Wells haftenden monoklonalen Kaninchen-Antikörper verschieden sind von dem Antikörper in dem anderen Well, und dass jedes der für eine Probe verwendeten Wells mit mindestens einem polyklonalen Anti-E7-Antikörper umgesetzt wird, wobei die polyklonalen Anti-E7-Antikörper, die mit der biologischen Probe umgesetzt werden, für jedes Well verschieden sind.

10. Diagnoseverfahren nach einem der Ansprüche 1 - 8, **dadurch gekennzeichnet, dass** in einem Testwell mindestens fünf verschiedene monoklonale Kaninchen-Antikörper, die an mindestens fünf verschiedene Epitope binden, als Fangantikörper verwendet werden, und wobei als Nachweisantikörper mindestens drei verschiedene polyklonale Anti-E7-Antikörper verwendet werden.

11. Diagnoseverfahren nach einem der Ansprüche 1 bis 10, **dadurch gekennzeichnet, dass** die biologische Probe von einem an Zervixkarzinom leidenden Patienten erhalten wird.

12. Diagnoseverfahren nach einem der Ansprüche 1 bis 10, **dadurch gekennzeichnet, dass** die biologische Probe von einem an Kopf- und Halskrebs leidenden Patienten erhalten wird.

13. Diagnoseverfahren nach einem der Ansprüche 1 bis 10, **dadurch gekennzeichnet, dass** die biologische Probe von einem an Analkarzinom leidenden Patienten erhalten wird.

14. Diagnose-Testkit zur Durchführung eines immunologischen Testverfahrens nach einem der Ansprüche 1 - 13, **dadurch gekennzeichnet, dass** es
eine feste Phase, beschichtet mit den in Anspruch 1 spezifizierten monoklonalen Kaninchen-Fangantikörpern,
Mittel zur Herstellung von Waschlösungen,
die in Anspruch 1 spezifizierten polyklonalen Nachweisantikörper und
Mittel zur Entwicklung eines Signals umfasst.

## Revendications

1. Procédé d'essai de diagnostic pour la détection d'une protéine E7 d'un papillomavirus humain dans un échantillon biologique **caractérisé par le fait que** dans un dosage immuno-enzymatique en sandwich (ELISA) sont utilisés, comme anticorps de capture, au moins trois anticorps monoclonaux de lapin qui se lient à au moins trois différents épitopes, et comme anticorps de détection, au moins deux anticorps anti-E7 polyclonaux différents qui sont obtenus par immunisation avec différents antigènes dérivés de différents types de HPV.

2. Procédé d'essai de diagnostic selon la revendication 1, **caractérisé par le fait qu'**au moins quatre anticorps monoclonaux de lapin qui se lient à au moins quatre épitopes différents sont utilisés comme anticorps de capture.

3. Procédé d'essai de diagnostic selon la revendication 1, **caractérisé par le fait qu'**au moins cinq anticorps monoclonaux de lapins qui se lient à au moins cinq épitopes différents sont utilisés comme anticorps de capture.

4. Procédé d'essai de diagnostic selon l'une quelconque des revendications 1 à 3, **caractérisé par le fait qu'**au moins trois anticorps anti-E7 polyclonaux différents sont utilisés comme anticorps de détection.

5. Procédé d'essai de diagnostic selon l'une quelconque des revendications 1 à 4, **caractérisé par le fait qu'**un anticorps polyclonal utilisé comme anticorps de détection a été obtenu par immunisation d'un animal avec HPV-16 E7.

6. Procédé d'essai de diagnostic selon l'une quelconque des revendications 1 à 4, **caractérisé par le fait qu'**un anticorps polyclonal utilisé comme anticorps de détection a été obtenu par immunisation d'un animal avec HPV-18 E7.

7. Procédé d'essai de diagnostic selon l'une quelconque des revendications 1 à 4, **caractérisé par le fait qu'**un anticorps polyclonal utilisé comme anticorps de détection a été obtenu par immunisation d'un animal avec un mélange des protéines E7 des HPV types 39, 51, 56 et 59.

8. Procédé d'essai de diagnostic selon l'une quelconque des revendications précédentes, **caractérisé par le fait que** les anticorps monoclonaux de lapin sont choisis dans le groupe comprenant des anticorps qui se lient aux épitopes situés dans les segments suivants d'acides aminés : 1-17 ; 11-37 ; 69-85 ; 31-51 ; 15-31 ; 85-98.

9. Procédé d'essai de diagnostic selon l'une quelconque des revendications 1 à 8, **caractérisé par le fait que** le même échantillon biologique est testé dans plusieurs puits de réaction, où chaque puits de réaction comprend au moins un anticorps monoclonal de lapin et les anticorps monoclonaux de lapin adhérant à chacun desdits puits est différent de l'anticorps dans l'autre puits et que chacun des puits utilisés pour un échantillon réagit avec au moins un anticorps anti-E7 polyclonal, où les anticorps anti-E7 polyclonaux qui réagissent avec ledit échantillon biologique sont différents pour chaque puits.

10. Procédé d'essai de diagnostic selon l'une quelconque des revendications 1 à 8, **caractérisé par le fait que** dans un puit de test au moins cinq anticorps monoclonaux de lapin différents qui se lient à au moins cinq épitopes différents sont utilisés comme anticorps de capture et où comme anticorps de détection au moins trois anticorps anti E7 polyclonaux différents sont utilisés.

11. Procédé d'essai de diagnostic selon l'une quelconque des revendications 1 à 10, **caractérisé par le fait que** l'échantillon biologique est obtenu à partir d'un patient souffrant d'un cancer du col de l'utérus.

12. Procédé d'essai de diagnostic selon l'une quelconque des revendications 1 à 10, **caractérisé par le fait que** l'échantillon biologique est obtenu à partir d'un patient souffrant d'un cancer de la tête et du cou.

13. Procédé d'essai de diagnostic selon l'une quelconque des revendications 1 à 10, **caractérisé par le fait que** l'échantillon biologique est obtenu à partir d'un patient souffrant d'un carcinome anal.

14. Kit de test de diagnostic pour la mise en oeuvre d'un procédé d'essai immunologique selon l'une quelconque des revendications 1 à 13, **caractérisé par le fait qu'**il comprend une phase solide revêtue des anticorps de capture monoclonaux de lapin spécifiés dans la revendication 1, des moyens de préparation de solutions de lavage, des anticorps de détection polyclonaux spécifiés dans la revendication 1 et des moyens de développement d'un signal.


### SEQUENCE LISTING

<110> Mikrogen GmbH
   Österreichische Akademie der Wissenschaften
<120> Immunological test for the detection of E7 oncoproteins in biological samples
<130> WO00250MIK
<150> EP14191193.3
   <151> 2014-10-31
<160> 101
<170> PatentIn version 3.5
<210> 1
   <211> 15
   <212> PRT
   <213> artificial sequence
<220>
   <223> 75-12
<400> 1
   Pro Glu Thr Thr Asp Leu Tyr Ser Tyr Glu Gln Leu Asn Asp Ser
   1               5                   10                  15
<210> 2
   <211> 15
   <212> PRT
   <213> artificial sequence
<220>
   <223> 75-12
<400> 2
   Pro Glu Pro Thr Asp Leu Tyr Ser Tyr Glu Gln Leu Ser Asp Ser
   1               5                   10                  15
<210> 3
   <211> 17
   <212> PRT
   <213> artificial sequence
<220>
   <223> 75-12
<400> 3
   Leu Glu Pro Glu Ala Thr Asp Leu Tyr Ser Tyr Glu Gln Leu Ser Asp
   1               5                   10                  15
   Ser
<210> 4
   <211> 13
   <212> PRT
   <213> artificial sequence
<220>
   <223> 75-12
<400> 4
   Pro Glu Thr Thr Asp Leu His Ser Tyr Glu Gln Leu Gly
   1               5                   10
<210> 5
   <211> 17
   <212> PRT
   <213> artificial sequence
<220>
   <223> 75-12
<400> 5
   Leu His Pro Glu Pro Thr Asp Leu Phe Ser Tyr Glu Gln Leu Ser Asp
   1               5                   10                  15
   Ser
<210> 6
   <211> 15
   <212> PRT
   <213> artificial sequence
<220>
   <223> consensus sequence
<220>
   <221> X
   <222> (1)..(15)
   <223> X is any amino acid
<400> 6
   Pro Glu Xaa Thr Asp Leu Xaa Ser Tyr Glu Gln Leu Xaa Asp Ser
   1               5                   10                  15
<210> 7
   <211> 15
   <212> PRT
   <213> artificial sequence
<220>
   <223> 75-12
<400> 7
   Asn Tyr Glu Glu Val Asp Leu Val Ser Tyr Glu Gln Leu Pro Asp
   1 5 10 15
<210> 8
   <211> 14
   <212> PRT
   <213> artificial sequence
<220>
   <223> 42-3
<400> 8
   Gly Thr Leu Gly Ile Val Ser Pro Ile Ser Ser Gln Lys Pro
   1 5 10
<210> 9
   <211> 17
   <212> PRT
   <213> artificial sequence
<220>
   <223> 143-7
<400> 9
   Met His Gly Lys Val Pro Thr Leu Gln Asp Val Val Leu Glu Leu Thr
   1 5 10 15
   Pro
<210> 10
   <211> 17
   <212> PRT
   <213> artificial sequence
<220>
   <223> 143-7
<400> 10
   Met His Gly Pro Lys Ala Thr Leu Gln Asp Ile Val Leu His Leu Glu
   1 5 10 15
   Pro
<210> 11
   <211> 15
   <212> PRT
   <213> artificial sequence
<220>
   <223> 143-7
<400> 11
   Gly Pro Lys Ala Thr Leu Gln Asp Ile Val Leu His Leu Glu Pro
   1 5 10 15
<210> 12
   <211> 15
   <212> PRT
   <213> artificial sequence
<220>
   <223> 143-7
<400> 12
   Met His Gly Pro Lys Ala Thr Leu Ser Asp Ile Val Leu Asp Leu
   1 5 10 15
<210> 13
   <211> 13
   <212> PRT
   <213> artificial sequence
<220>
   <223> 143-7
<400> 13
   Asn Pro Thr Leu Arg Glu Tyr Ile Leu Asp Leu His Pro
   1 5 10
<210> 14
   <211> 17
   <212> PRT
   <213> artificial sequence
<220>
   <223> consensus sequence
<220>
   <221> X
   <222> (1)..(17)
   <223> X is any amino acid
<400> 14
   Met His Gly Xaa Xaa Xaa Thr Leu Xaa Xaa Xaa Xaa Leu Xaa Leu Xaa
   1 5 10 15
   Pro
<210> 15
   <211> 19
   <212> PRT
   <213> artificial sequence
<220>
   <223> 21-10
<400> 15
   Leu Asp Leu Gln Pro Glu Thr Thr Asp Leu Tyr Ser Tyr Glu Gln Leu
   1               5                   10                  15
   Asn Asp Ser
<210> 16
   <211> 21
   <212> PRT
   <213> artificial sequence
<220>
   <223> 21-10
<400> 16
   Tyr Val Leu Asp Leu Gln Pro Lys Ala Thr Asp Leu His Ser Tyr Glu
   1               5                   10                  15
   Gln Leu Pro Asp Ser
               20
<210> 17
   <211> 17
   <212> PRT
   <213> artificial sequence
<220>
   <223> 21-10
<400> 17
   Leu Asp Leu Tyr Pro Glu Pro Thr Asp Leu Tyr Ser Tyr Glu Gln Leu
   1               5                   10                  15
   Ser
<210> 18
   <211> 15
   <212> PRT
   <213> artificial sequence
<220>
   <223> 21-10
<400> 18
   Leu Asp Leu Glu Pro Glu Ala Thr Asp Leu Tyr Ser Tyr Glu Gln
   1               5                   10                  15
<210> 19
   <211> 21
   <212> PRT
   <213> artificial sequnece
<220>
   <223> 21-10
<400> 19
   Tyr Ile Leu Asp Leu Gln Pro Glu Thr Thr Asp Leu His Ser Tyr Glu
   1               5                   10                  15
   Gln Leu Gly Asp Ser
               20
<210> 20
   <211> 17
   <212> PRT
   <213> artificial sequence
<220>
   <223> 21-10
<400> 20
   Leu Asp Leu His Pro Glu Pro Thr Asp Leu Phe Ser Tyr Glu Gln Leu
   1               5                   10                  15
   Ser
<210> 21
   <211> 17
   <212> PRT
   <213> artificial sequence
<220>
   <223> consensus sequence
<220>
   <221> X
   <222> (1)..(12)
   <223> X is any amino acid
<220>
   <221> misc_feature
   <222> (17)..(17)
   <223> Xaa can be any naturally occurring amino acid
<400> 21
   Leu Asp Leu Xaa Pro Xaa Xaa Thr Asp Leu Xaa Ser Tyr Glu Gln Leu
   1               5                   10                  15
   Xaa
<210> 22
   <211> 19
   <212> PRT
   <213> artificial sequence
<220>
   <223> 57-4
<400> 22
   Leu Asp Leu Gln Pro Glu Thr Thr Asp Leu Tyr Ser Tyr Glu Gln Leu
   1               5                   10                  15
   Asn Asp Ser
<210> 23
   <211> 17
   <212> PRT
   <213> artificial sequence
<220>
   <223> 57-4
<400> 23
   Leu Gln Pro Lys Ala Thr Asp Leu His Ser Tyr Glu Gln Leu Pro Asp
   1               5                   10                  15
   Ser
<210> 24
   <211> 17
   <212> PRT
   <213> artificial sequence
<220>
   <223> 57-4
<400> 24
   Leu Asp Leu Tyr Pro Glu Pro Thr Asp Leu Tyr Ser Tyr Glu Gln Leu
   1               5                   10                  15
   Ser
<210> 25
   <211> 19
   <212> PRT
   <213> artificial sequence
<220>
   <223> 57-4
<400> 25
   Leu Asp Leu Glu Pro Glu Ala Thr Asp Leu Tyr Ser Tyr Glu Gln Leu
   1 5 10 15
   Ser Asp Ser
<210> 26
   <211> 17
   <212> PRT
   <213> artificial sequence
<220>
   <223> 57-4
<400> 26
   Leu Gln Pro Glu Thr Thr Asp Leu His Ser Tyr Glu Gln Leu Gly Asp
   1 5 10 15
   Ser
<210> 27
   <211> 19
   <212> PRT
   <213> artificial sequence
<220>
   <223> 57-4
<400> 27
   Leu Asp Leu His Pro Glu Pro Thr Asp Leu Phe Ser Tyr Glu Gln Leu
   1 5 10 15
   Ser Asp Ser
<210> 28
   <211> 19
   <212> PRT
   <213> artificial sequence
<220>
   <223> consensus sequence
<220>
   <221> X
   <222> (1)..(14)
   <223> X is any amino acid
<220>
   <221> misc_feature
   <222> (17)..(17)
   <223> Xaa can be any naturally occurring amino acid
<400> 28
   Leu Asp Leu Xaa Pro Xaa Xaa Thr Asp Leu Xaa Ser Tyr Glu Gln Leu
   1 5 10 15
   Xaa Asp Ser
<210> 29
   <211> 15
   <212> PRT
   <213> artificial sequence
<220>
   <223> 57-4
<400> 29
   Glu Gln Leu Asp Ser Ser Glu Asp Glu Asp Glu Asp Glu Val Asp
   1 5 10 15
<210> 30
   <211> 19
   <212> PRT
   <213> artificial sequence
<220>
   <223> 58-3
<400> 30
   Leu Asp Leu Gln Pro Glu Thr Thr Asp Leu Tyr Ser Tyr Glu Gln Leu
   1 5 10 15
   Asn Asp Ser
<210> 31
   <211> 17
   <212> PRT
   <213> artificial sequence
<220>
   <223> 58-3
<400> 31
   Leu Gln Pro Lys Ala Thr Asp Leu His Ser Tyr Glu Gln Leu Pro Asp
   1 5 10 15
   Ser
<210> 32
   <211> 21
   <212> PRT
   <213> artificial sequence
<220>
   <223> 58-3
<400> 32
   Tyr Val Leu Asp Leu Tyr Pro Glu Pro Thr Asp Leu Tyr Ser Tyr Glu
   1               5                   10                  15
   Gln Leu Ser Asp Ser
               20
<210> 33
   <211> 17
   <212> PRT
   <213> artificial sequence
<220>
   <223> 58-3
<400> 33
   Leu Glu Pro Glu Ala Thr Asp Leu Tyr Ser Tyr Glu Gln Leu Ser Asp
   1               5                   10                  15
   Ser
<210> 34
   <211> 15
   <212> PRT
   <213> artificial sequence
<220>
   <223> 58-3
<400> 34
   Leu Gln Pro Glu Thr Thr Asp Leu His Ser Tyr Glu Gln Leu Gly
   1               5                   10                  15
<210> 35
   <211> 19
   <212> PRT
   <213> artificial sequence
<220>
   <223> 58-3
<400> 35
   Leu Asp Leu His Pro Glu Pro Thr Asp Leu Phe Ser Tyr Glu Gln Leu
   1               5                   10                  15
   Ser Asp Ser
<210> 36
   <211> 19
   <212> PRT
   <213> artificial sequence
<220>
   <223> consensus sequence
<220>
   <221> X
   <222> (1)..(14)
   <223> X is any amino acid
<220>
   <221> misc_feature
   <222> (17)..(17)
   <223> Xaa can be any naturally occurring amino acid
<400> 36
   Leu Asp Leu Xaa Pro Xaa Xaa Thr Asp Leu Xaa Ser Tyr Glu Gln Leu
   1               5                   10                  15
   Xaa Asp Ser
<210> 37
   <211> 15
   <212> PRT
   <213> artificial sequence
<220>
   <223> 58-3
<400> 37
   Glu Gln Leu Asp Ser Ser Glu Asp Glu Asp Glu Asp Glu Val Asp
   1               5                   10                  15
<210> 38
   <211> 13
   <212> PRT
   <213> artificial sequence
<220>
   <223> 78-11
<400> 38
   Met Arg Gly Asn Asn Pro Thr Leu Arg Glu Tyr Ile Leu
   1               5                   10
<210> 39
   <211> 15
   <212> PRT
   <213> artificial sequence
<220>
   <223> 80-2
<400> 39
   Glu Glu Glu Asp Glu Ile Asp Gly Pro Ala Gly Gln Ala Glu Pro
   1 5 10 15
<210> 40
   <211> 13
   <212> PRT
   <213> artificial sequence
<220>
   <223> 80-2
<400> 40
   Val Ile Asp Ser Pro Ala Gly Gln Ala Lys Pro Asp Thr
   1 5 10
<210> 41
   <211> 11
   <212> PRT
   <213> artificial sequence
<220>
   <223> consensus sequence
<220>
   <221> X
   <222> (1)..(11)
   <223> X is any amino acid
<400> 41
   Xaa Ile Asp Xaa Pro Ala Gly Gln Ala Xaa Pro
   1 5 10
<210> 42
   <211> 13
   <212> PRT
   <213> artificial sequence
<220>
   <223> 80-2
<400> 42
   Met Asp Thr Leu Ser Phe Val Ser Pro Leu Ser Ala Ala
   1 5 10
<210> 43
   <211> 15
   <212> PRT
   <213> artificial sequence
<220>
   <223> 84-2
<400> 43
   Glu Asp Val Ile Asp Ser Pro Ala Gly Gln Ala Lys Pro Asp Thr
   1 5 10 15
<210> 44
   <211> 19
   <212> PRT
   <213> artificial sequence
<220>
   <223> 84-2
<400> 44
   Ser Ser Asp Glu Asp Glu Gly Leu Asp Arg Pro Asp Gly Gln Ala Gln
   1 5 10 15
   Pro Ala Thr
<210> 45
   <211> 19
   <212> PRT
   <213> artificial sequence
<220>
   <223> 84-2
<400> 45
   Asp Glu Glu Asp Thr Asp Gly Val Asp Arg Pro Asp Gly Gln Ala Glu
   1 5 10 15
   Gln Ala Thr
<210> 46
   <211> 21
   <212> PRT
   <213> artificial sequence
<220>
   <223> 84-2
<400> 46
   Ser Ser Asp Glu Asp Glu Ile Gly Leu Asp Arg Pro Asp Gly Gln Ala
   1 5 10 15
   Gln Pro Ala Thr Ala
   20
<210> 47
   <211> 20
   <212> PRT
   <213> artificial sequence
<220>
   <223> consensus sequence
<220>
   <221> X
   <222> (1)..(20)
   <223> X is any amino acid
<400> 47
   Ser Xaa Xaa Glu Asp Xaa Xaa Xaa Xaa Asp Arg Pro Xaa Gly Gln Ala
   1               5                   10                  15
   Xaa Xaa Ala Thr
               20
<210> 48
   <211> 13
   <212> PRT
   <213> artificial sequence
<220>
   <223> 19-1
<400> 48
   Val Pro Thr Leu Gln Asp Val Val Leu Glu Leu Thr Pro
   1               5                   10
<210> 49
   <211> 13
   <212> PRT
   <213> artificial sequence
<220>
   <223> 19-1
<400> 49
   Met His Gly Pro Lys Ala Thr Leu Ser Asp Ile Val Leu
   1               5                   10
<210> 50
   <211> 9
   <212> PRT
   <213> artificial sequence
<220>
   <223> consensus sequence
<220>
   <221> X
   <222> (1) .. (9)
   <223> X is any amino acid
<400> 50
   Xaa Xaa Thr Leu Xaa Asp Xaa Val Leu
   1 5
<210> 51
   <211> 15
   <212> PRT
   <213> artificial sequence
<220>
   <223> 31-7
<400> 51
   Glu Gln Leu Glu Asp Ser Ser Glu Asp Glu Val Asp Lys Val Lys
   1 5 10 15
<210> 52
   <211> 21
   <212> PRT
   <213> artificial sequence
<220>
   <223> 31-7
<400> 52
   Gln Leu Asn Asp Ser Ser Glu Glu Glu Asp Glu Ile Asp Gly Pro Ala
   1 5 10 15
   Gly Gln Ala Glu Pro
   20
<210> 53
   <211> 23
   <212> PRT
   <213> artificial sequence
<220>
   <223> 31-7
<400> 53
   Glu Gln Leu Ser Asp Ser Glu Glu Glu Asn Asp Glu Ile Asp Gly Val
   1 5 10 15
   Asn His Gln His Leu Pro Ala
   20
<210> 54
   <211> 17
   <212> PRT
   <213> artificial sequence
<220>
   <223> 31-7
<400> 54
   Ser Ser Asp Glu Glu Asp Val Ile Asp Ser Pro Ala Gly Gln Ala Lys
   1               5                   10                  15
   Pro
<210> 55
   <211> 24
   <212> PRT
   <213> artificial sequence
<220>
   <223> consensus
<220>
   <221> X
   <222> (1)..(24)
   <223> X is any amino acid
<400> 55
   Glu Gln Leu Xaa Asp Ser Ser Xaa Glu Glu Xaa Asp Glu Val Ile Asp
   1               5                   10                  15
   Xaa Xaa Xaa Xaa Gln Xaa Xaa Pro
               20
<210> 56
   <211> 19
   <212> PRT
   <213> artificial sequence
<220>
   <223> 31-7
<400> 56
   Ser Ser Glu Asp Glu Asp Glu Asp Glu Val Asp His Leu Gln Glu Arg
   1               5                   10                  15
   Pro Gln Gln
<210> 57
   <211> 23
   <212> PRT
   <213> artificial sequence
<220>
   <223> 31-7
<400> 57
   Val Ser His Glu Gln Leu Gly Asp Ser Asp Asp Glu Ile Asp Glu Pro
   1 5 10 15
   Asp His Ala Val Asn His His
   20
<210> 58
   <211> 13
   <212> PRT
   <213> artificial sequence
<220>
   <223> 55-11
<400> 58
   Leu Glu Asp Ser Ser Glu Asp Glu Val Asp Lys Val Asp
   1 5 10
<210> 59
   <211> 17
   <212> PRT
   <213> artificial sequence
<220>
   <223> 55-11
<400> 59
   Asp Ser Glu Glu Glu Asn Asp Glu Ile Asp Gly Val Asn His Gln His
   1 5 10 15
   Leu
<210> 60
   <211> 15
   <212> PRT
   <213> artificial sequence
<220>
   <223> consensus sequence
<220>
   <221> X
   <222> (1)..(15)
   <223> X is any amino acid
<400> 60
   Asp Ser Xaa Glu Xaa Xaa Xaa Asp Glu Xaa Xaa Asp Xaa Val Xaa
   1 5 10 15
<210> 61
   <211> 21
   <212> PRT
   <213> artificial sequence
<220>
   <223> epitope
<400> 61
   Leu His Leu Glu Pro Gln Asn Glu Leu Asp Pro Val Asp Leu Leu Ser
   1 5 10 15
   Tyr Glu Gln Leu Ser
   20
<210> 62
   <211> 19
   <212> PRT
   <213> artificial sequence
<220>
   <223> epitope
<400> 62
   Leu Glu Pro Gln Asn Tyr Glu Glu Val Asp Leu Val Ser Tyr Glu Gln
   1 5 10 15
   Leu Pro Asp
<210> 63
   <211> 21
   <212> PRT
   <213> artificial sequence
<220>
   <223> epitope
<400> 63
   Leu Glu Leu Ser Pro Ser Asn Glu Ile Glu Pro Val Asp Leu Val Ser
   1 5 10 15
   His Glu Gln Leu Gly
   20
<210> 64
   <211> 13
   <212> PRT
   <213> artificial sequence
<220>
   <223> epitope
<400> 64
   Leu Tyr Pro Tyr Asn Glu Ile Gln Pro Val Asp Leu Val
   1 5 10
<210> 65
   <211> 22
   <212> PRT
   <213> artificial sequence
<220>
   <223> consensus
<220>
   <221> X
   <222> (1)..(22)
   <223> X is any amino acid
<400> 65
   Leu Xaa Leu Xaa Pro Xaa Asn Glu Xaa Xaa Xaa Val Asp Leu Xaa Ser
   1               5                   10                  15
   Xaa Glu Gln Leu Xaa Xaa
               20
<210> 66
   <211> 13
   <212> PRT
   <213> artificial sequence
<220>
   <223> epitope
<400> 66
   Phe Ser Ser Gln Ser Glu Ser Thr Leu Arg Leu Ser Val
   1               5                   10
<210> 67
   <211> 13
   <212> PRT
   <213> artificial sequence
<220>
   <223> epitope
<400> 67
   Met His Gly Arg Leu Val Thr Leu Lys Asp Ile Val Leu
   1               5                   10
<210> 68
   <211> 17
   <212> PRT
   <213> artificial sequence
<220>
   <223> epitope
<400> 68
   Met His Gly Lys Val Pro Thr Leu Gln Asp Val Val Leu Glu Leu Thr
   1               5                   10                  15
   Pro
<210> 69
   <211> 13
   <212> PRT
   <213> artificial sequence
<220>
   <223> consensus sequence
<220>
   <221> X
   <222> (1)..(13)
   <223> X is any amino acid
<400> 69
   Met His Gly Xaa Xaa Xaa Thr Leu Xaa Asp Xaa Val Leu
   1               5                   10
<210> 70
   <211> 13
   <212> PRT
   <213> artificial sequence
<220>
   <223> epitope
<400> 70
   Pro Leu Thr Gln His Tyr Gln Ile Leu Thr Ser Ser Ser
   1               5                   10
<210> 71
   <211> 13
   <212> PRT
   <213> artificial sequence
<220>
   <223> epitope
<400> 71
   Arg Ala His Tyr Asn Ile Val Thr Phe Ser Ser Lys Ser
   1               5                   10
<210> 72
   <211> 13
   <212> PRT
   <213> artificial sequence
<220>
   <223> epitope
<400> 72
   Thr Ser Asn Tyr Asn Ile Val Thr Phe Ser Ser Gln Ser
   1               5                   10
<210> 73
   <211> 13
   <212> PRT
   <213> artificial sequence
<220>
   <223> epitope
<400> 73
   Ala Thr Ala Asn Tyr Tyr Ile Val Thr Ser Ser Tyr Thr
   1               5                   10
<210> 74
   <211> 13
   <212> PRT
   <213> artificial sequence
<220>
   <223> consensus
<220>
   <221> X
   <222> (1)..(13)
   <223> X is any amino acid
<400> 74
   Xaa Xaa Xaa Tyr Xaa Ile Xaa Thr Phe Ser Ser Xaa Xaa
   1               5                   10
<210> 75
   <211> 15
   <212> PRT
   <213> artificial sequence
<220>
   <223> epitope
<400> 75
   Leu Asn Thr Leu Ser Phe Val Ser Pro Trp Ser Ala Ser Gln Gln
   1               5                   10                  15
<210> 76
   <211> 13
   <212> PRT
   <213> artificial sequence
<220>
   <223> epitope
<400> 76
   Thr Leu Ser Phe Val Ser Pro Trp Ser Ala Thr Asn Gln
   1 5 10
<210> 77
   <211> 13
   <212> PRT
   <213> artificial sequence
<220>
   <223> epitope
<400> 77
   Ala Leu Thr Val Thr Ser Pro Leu Ser Ala Ser Ser Asn
   1 5 10
<210> 78
   <211> 13
   <212> PRT
   <213> artificial sequence
<220>
   <223> consensus sequence
<220>
   <221> X
   <222> (1)..(13)
   <223> X is any amino acid
<400> 78
   Xaa Leu Xaa Xaa Xaa Ser Pro Xaa Ser Ala Xaa Xaa Xaa
   1 5 10
<210> 79
   <211> 13
   <212> PRT
   <213> artificial sequence
<220>
   <223> epitope
<400> 79
   Asp Arg Pro Asp Gly Gln Ala Glu Gln Ala Thr Ser Asn
   1 5 10
<210> 80
   <211> 19
   <212> PRT
   <213> artificial sequence
<220>
   <223> epitope
<400> 80
   Met His Gly Arg Leu Val Thr Leu Lys Asp Ile Val Leu Asp Leu Gln
   1               5                   10                  15
   Pro Pro Asp
<210> 81
   <211> 18
   <212> PRT
   <213> Artificial Sequence
<220>
   <223> epitope
<400> 81
   Gly Pro Lys Ala Thr Leu Gln Asp Ile Val Leu His Leu Leu Glu Pro
   1               5                   10                  15
   Gln Asn
<210> 82
   <211> 21
   <212> PRT
   <213> artificial sequence
<220>
   <223> epitope
<400> 82
   Met His Gly Lys Val Pro Thr Leu Gln Asp Val Val Leu Glu Leu Thr
   1               5                   10                  15
   Pro Gln Thr Glu Ile
               20
<210> 83
   <211> 15
   <212> PRT
   <213> artificial sequence
<220>
   <223> epitope
<400> 83
   Met His Gly Pro Lys Ala Thr Leu Ser Asp Ile Val Leu Asp Leu
   1               5                   10                  15
<210> 84
   <211> 20
   <212> PRT
   <213> artificial sequence
<220>
   <223> consensus sequence
<220>
   <221> X
   <222> (1)..(20)
   <223> X is any amino acid
<400> 84
   Met His Gly Xaa Xaa Xaa Thr Leu Xaa Asp Xaa Val Xaa Leu Leu Xaa
   1               5                   10                  15
   Xaa Xaa Xaa Xaa
               20
<210> 85
   <211> 13
   <212> PRT
   <213> artificial sequence
<220>
   <223> epitope
<400> 85
   Tyr Ser Tyr Glu Gln Leu Asn Asp Ser Ser Glu Glu Glu
   1               5                   10
<210> 86
   <211> 15
   <212> PRT
   <213> artificial sequence
<220>
   <223> epitope
<400> 86
   Leu Gln Pro Lys Ala Thr Asp Leu His Ser Tyr Glu Gln Leu Pro
   1               5                   10                  15
<210> 87
   <211> 27
   <212> PRT
   <213> artificial sequence
<220>
   <223> epitope
<400> 87
   Leu Asp Leu Tyr Pro Glu Pro Thr Asp Leu Tyr Ser Tyr Glu Gln Leu
   1 5 10 15
   Ser Asp Ser Ser Asp Glu Asp Glu Gly Leu Asp
   20 25
<210> 88
   <211> 17
   <212> PRT
   <213> artificial sequence
<220>
   <223> epitope
<400> 88
   Leu Glu Pro Glu Ala Thr Asp Leu Tyr Ser Tyr Glu Gln Leu Ser Asp
   1 5 10 15
   Ser
<210> 89
   <211> 21
   <212> PRT
   <213> artificial sequence
<220>
   <223> consensus sequence
<220>
   <221> X
   <222> (1)..(21)
   <223> X is any amino acid
<400> 89
   Leu Xaa Pro Xaa Xaa Thr Asp Leu Xaa Ser Tyr Glu Gln Leu Xaa Asp
   1 5 10 15
   Ser Ser Xaa Glu Xaa
   20
<210> 90
   <211> 19
   <212> PRT
   <213> artificial sequence
<220>
   <223> epitope
<400> 90
   Met His Gly Arg Leu Val Thr Leu Lys Asp Ile Val Leu Asp Leu Gln
   1 5 10 15
   Pro Pro Asp
<210> 91
   <211> 19
   <212> PRT
   <213> artificial sequence
<220>
   <223> epitope
<400> 91
   Met His Gly Pro Lys Ala Thr Leu Gln Asp Ile Val Leu His Leu Glu
   1 5 10 15
   Pro Gln Asn
<210> 92
   <211> 19
   <212> PRT
   <213> artificial sequence
<220>
   <223> epitope
<400> 92
   Met His Gly Pro Gln Ala Thr Leu Gln Glu Ile Val Leu His Leu Glu
   1 5 10 15
   Pro Gln Asn
<210> 93
   <211> 23
   <212> PRT
   <213> artificial sequence
<220>
   <223> epitope
<400> 93
   Met His Gly Lys Val Pro Thr Leu Gln Asp Val Val Leu Glu Leu Thr
   1 5 10 15
   Pro Gln Thr Glu Ile Asp Leu
   20
<210> 94
   <211> 15
   <212> PRT
   <213> artificial sequence
<220>
   <223> epitope
<400> 94
   Met His Gly Pro Lys Ala Thr Leu Ser Asp Ile Val Leu Asp Leu
   1 5 10 15
<210> 95
   <211> 13
   <212> PRT
   <213> artificial sequence
<220>
   <223> epitope
<400> 95
   Thr Leu Gln Glu Ile Val Leu Asp Leu Tyr Pro Tyr Asn
   1 5 10
<210> 96
   <211> 19
   <212> PRT
   <213> artificial sequence
<220>
   <223> consensus sequence
<220>
   <221> X
   <222> (1)..(19)
   <223> X is any amino acid
<400> 96
   Met His Gly Xaa Xaa Xaa Thr Leu Xaa Xaa Xaa Val Leu Xaa Leu Xaa
   1 5 10 15
   Pro Xaa Xaa
<210> 97
   <211> 19
   <212> PRT
   <213> artificial sequence
<220>
   <223> epitope
<400> 97
   Met His Gly Arg Leu Val Thr Leu Lys Asp Ile Val Leu Asp Leu Gln
   1 5 10 15
   Pro Pro Asp
<210> 98
   <211> 17
   <212> PRT
   <213> artificial sequence
<220>
   <223> epitope
<400> 98
   Gly Pro Lys Ala Thr Leu Gln Asp Ile Val Leu His Leu Glu Pro Gln
   1               5                   10                  15
   Asn
<210> 99
   <211> 23
   <212> PRT
   <213> artificial sequence
<220>
   <223> epitope
<400> 99
   Met His Gly Lys Val Pro Thr Leu Gln Asp Val Val Leu Glu Leu Thr
   1               5                   10                  15
   Pro Gln Thr Glu Ile Asp Leu
               20
<210> 100
   <211> 15
   <212> PRT
   <213> artificial sequence
<220>
   <223> epitope
<400> 100
   Met His Gly Pro Lys Ala Thr Leu Ser Asp Ile Val Leu Asp Leu
   1               5                   10                  15
<210> 101
   <211> 19
   <212> PRT
   <213> artificial sequence
<220>
   <223> consensus sequence
<220>
   <221> X
   <222> (1) .. (19)
   <223> X is any amino acid
<400> 101
   Met His Gly Xaa Xaa Xaa Thr Leu Xaa Asp Xaa Val Leu Xaa Leu Xaa
   1               5                   10                  15
   Pro Xaa Xaa